# FASCICULE DE BREVET EUROPEEN

(11) **EP 3 187 046 B1**
(45) Date de publication et mention de la délivrance du brevet: **12.12.2018**
(21) Numéro de dépôt: 16205679.0
(22) Date de dépôt: 21.12.2016
(51) Int. Cl.: A01N 25/18, A01N 25/10, A01N 35/02, A01N 37/02, A01N 37/04, A01N 43/90, A01N 31/02, A01P 19/00, A01P 15/00, A61K 9/00, A61K 47/32

(54) **COMPOSITION SOLIDE POUR LE RELARGAGE CONTROLE DE SUBSTANCES SEMIOCHIMIQUES**
FESTE ZUSAMMENSETZUNG ZUR KONTROLLIERTEN FREISETZUNG VON SEMIOCHEMIKALIEN
SOLID COMPOSITION FOR THE DELIVERY OF SEMIOCHEMICAL SUBSTANCES

(30) Priorité: 31.12.2015 FR 1563491
(43) Date de publication de la demande: 05.07.2017
(73) Titulaire: Melchior Material And Life Science France, 64170 Lacq (FR)
(72) Inventeur: GUERRET, Olivier, 46170 PERN (FR); DUFOUR, Samuel, 64300 ORTHEZ (FR)
(74) Mandataire: Regimbeau

(56) Documents cités:
- Arkema: "Pebax - Polyether - Block - Amides", , 26 août 2009 (2009-08-26), pages 1-14, XP055196407, Extrait de l'Internet: URL:http://www.pebax.com/export/sites/peba x/.content/medias/downloads/literature/peb ax-product-range-brochure.pdf [extrait le 2015-06-17]
- Jean-Pierre Disson ET AL: "Les Polymères Nanostructurés d'Arkema", Colloque Nanomatériaux, 22 septembre 2009 (2009-09-22), pages 1-18, XP055264793, Paris Extrait de l'Internet: URL:http://cmm.mines-paristech.fr/Nanomine s/Rapports/2009/presentation_lebouvier.pdf [extrait le 2016-04-12]
- H.E. Hummel ET AL: "Dreams versus fulfilled realities in pheromone technologies, and their recent viticultural applications towards sustainable Lobesia management", , 22 octobre 2015 (2015-10-22), pages 1-54, XP055264702, Extrait de l'Internet: URL:https://www.iobc-wprs.org/pub/2015_WG_ Viticulture_Vienna/20151022_00_IOBC_Vienna _Entomology_Hummel.pdf [extrait le 2016-04-12]
- L: "Grape Berry Moth Pheromone - Decision Document E92-03", , 22 décembre 1992 (1992-12-22), pages 1-5, XP055264730, Ottawa Extrait de l'Internet: URL:http://publications.gc.ca/collections/ Collection/H113-4-92-03E.pdf [extrait le 2016-04-12]
- CARDE R T ET AL: "CONTROL OF MOTH PESTS BY MATING DISRUPTION: SUCCESSES AND CONSTRAINTS", ANNUAL REVIEW OF ENTOMOLOGY, XX, XX, vol. 40, 1 janvier 1995 (1995-01-01), pages 559-585, XP001037740, DOI: 10.1146/ANNUREV.EN.40.010195.003015

## Description

Les substances sémiochimiques (phéromones, kairomones, allomones et synomones), sont des substances volatiles de plus en plus utilisées dans de nombreux domaines que ce soit, par exemple, pour contrôler le comportement des animaux ou pour réguler les populations d'insectes ravageurs. Par ailleurs, ces substances sémiochimiques sont des molécules sensibles chimiquement et plus particulièrement sensibles à l'oxydation (notamment en présence d'UV ou de radicaux libres) ou aux hautes températures car présentant des liaisons chimiques insaturées (double ou triple liaisons), des groupes alcool ou des cycles éther tendus. Or la formation d'impuretés par des processus d'oxydation peut avoir des effets catastrophiques sur l'efficacité des sémiochimiques (un attractant peut par exemple devenir un répulsif ou bien la phéromone d'un insecte en particulier peut se transformer en la phéromone d'un autre insecte).

Un des problèmes majeur qui se pose pour pouvoir utiliser de manière optimale ces substances sémiochimiques est la possibilité de les stocker et de les relarguer à l'aide de dispositifs ou de matériaux permettant non seulement la préservation de la pureté des sémiochimiques pendant la fabrication du dispositif et pendant le temps d'utilisation, mais aussi un relargage contrôlé à des taux suffisants sur de longues périodes.

La présente invention propose donc un nouveau type de composition solide rechargeable constituée d'une matrice polymérique et d'une substance sémiochimique permettant d'atteindre ce triple objectif d'adsorption, de protection et de relargage contrôlé de ladite substance sur de longues durées.

### ART ANTÉRIEUR

### Champ de l'invention :

Cette invention se rapporte au relargage contrôlé dans l'atmosphère de substances sémiochimiques variées par une composition solide comportant une matrice polymérique qui est solide à température ambiante et une substance sémiochimique incorporée dans ladite matrice.

Plus particulièrement et afin d'éviter la détérioration de la substance sémiochimique, la matrice polymérique doit permettre l'incorporation de la substance sémiochimique sans que cette substance ne soit altérée par un processus de polymérisation, ou par l'utilisation de températures élevées.

De plus, afin d'éviter la présence de volatils qui interagiraient négativement avec les animaux, l'usage de solvants organiques pour faciliter l'adsorption de diverses substances sémiochimiques dans la matrice doit être évitée. Indépendamment, de l'effet de tels solvants sur les animaux, l'impact environnemental de tels solvants rend préférable de s'en affranchir.

### Description de l'art antérieur :

L'utilisation de matrices polymériques pour le relargage de substances actives, notamment des substances sémiochimiques est connue depuis longtemps puisque déjà évoquée par exemple dans la revue de Campion (D.G. Campion, Pestic. Sci. 1978, 9, 434-440). Il est notamment fait référence dans cette publication à l'utilisation de matrices caoutchouteuses (ou élastomériques). Cependant, il y est aussi souligné que de telles matrices ne permettent pas un relargage continu dans le temps et présentent une grande sensibilité aux conditions atmosphériques.

Plus tard, d'autres matrices élastomériques ont été proposées : par exemple, en 1987, dans le brevet US4703070. Mais, ces matrices élastomériques nécessitent d'incorporer la substance active avant que la matrice élastomérique ne soit totalement réticulée. Or, à cause de leurs structures chimiques spécifiques (liaison insaturées, présence de fonctions alcool...) peu de substances sémiochimiques peuvent supporter sans détérioration soit les hautes températures nécessaires à la fin de la réticulation, soit les composants réactifs du pré-polymère. Or l'activité des substances sémiochimiques est fortement influencée par une détérioration même légère de la molécule (comme un changement d'isomérie par exemple).

Ultérieurement, l'idée d'utiliser des matrices polymériques à base de copolymères pour relarguer des produits volatiles est apparue. Par exemple dans le brevet de Lee déposé en 1989, (US 5008115A) est décrite une matrice polymérique, pour délivrer des substances actives (parfums, phéromones...), formée d'un bloc polymère « mou » et d'un bloc polymère « dur ». L'un des deux copolymères doit être perméable à la substance active. Dans ce brevet, les segments durs sont des polyuréthanes ou des polyurées dérivés d'un isocyanate organique. C'est à cause des isocyanates que ce type particulier de matrice de copolymère est incompatible avec une majorité de substances actives sémiochimiques. En effet, ces dernières possèdent généralement des fonctions chimiques qui vont réagir avec les isocyanates (liaisons insaturées, fonctions alcool...). Par exemple, la phéromone de la sésamie du maïs (mélange de Z11 hexadécénol et de Z11 hexadécényl acetate) se dégrade en présence d'isocyanate, de même que le ferruginéol (4-méthyl 5-nonanol), phéromone du charançon du palmier dattier. Enfin, la présence d'isocyanates résiduels hautement toxiques est aussi un inconvénient majeur de ce type de matrice.

Une autre matrice thermoplastique pour le relargage de sémiochimiques a fait l'objet d'un dépôt de brevet en 1996 (US005504142A). Dans ce cas, la matrice est un copolymère de type PEBAX®. Le Pebax® est un élastomère thermoplastique ou un polyamide flexible sans plastifiant composé d'une chaîne linéaire régulière de segments polyamides rigides et de segments polyéther flexibles. Cependant ce type de matrice nécessite l'utilisation d'un solvant servant de vecteur à la substance active pour pénétrer dans la matrice copolymère. Ces solvants nécessaires à la mise en oeuvre du procédé décrit dans cette invention sont potentiellement nocifs pour l'environnement ou peuvent avoir une interaction négative avec les animaux. La présence de tels solvants (dérivés de l'acide undécylénique aux propriétés biocides bien connues de l'homme du métier) est donc rédhibitoire pour une utilisation d'une telle matrice pour stocker et relarguer des substances sémiochimiques.

Une autre grande famille de matrices copolymères a ensuite été proposée à base de polyéthylène glycol (PEG). En 2005 par exemple, Brown et al (J. Am. Chem. Soc. 2005, 127, 11238-11239), proposaient d'utiliser un copolymère à base de polyéthylène glycol (PEG) et d'un fluoropolymère hydrophobe hyperbranché pour stocker et relarguer un alcool terpénique insaturé (le géraniol). Bien que montrant de grandes capacités à stocker cette molécule, un tel copolymère conduisait à des temps de relargage de la substance active très réduits ce qui est incompatible avec les relargages sur des longues durées nécessaires dans l'utilisation des sémiochimiques dans l'agriculture. En 2010, Shakil et al (p228 de la revue de M. Kah dans Environment International 63 (2014) 224-235) proposaient des formulations aqueuses à base de copolymères amphiphiles contenant des blocs PEG pour relarguer des insecticides. Cependant ces formulations aqueuses présentent l'inconvénient de ne pas être solides et de la présence d'eau peut induire des modifications chimiques des sémiochimiques.

Les matrices copolymères d'éthylène et de vinyl acétate ont aussi été testées pour délivrer des composés bioactifs tels que des attractants alimentaires, des pesticides, comme décrit dans le brevet US5135744A. Cependant, le mélange du copolymère et du composé bioactif et la mise en forme finale (extrusion) doivent se faire à des températures élevées (entre 80 et 110°C) ce qui n'est pas réalisable avec la plupart des substances sémiochimiques qui soit ne supportent pas ces conditions de transformation soit s'évaporent rapidement à la sortie de l'extrudeuse ce qui rend la charge de phéromone dans le plastiques difficilement contrôlable.

D'autres copolymères ont aussi été proposés par exemple dans WO2012/095444, qui revendique la formation de microcapsules composées de plusieurs polymères dont des C1-C24-alkyl ester d'acide acrylique. Ce type de matrice ne peut être utilisé que pour des sémiochimiques ne présentant pas de sensibilité aux peroxydes qui sont nécessaires à la polymérisation de la matrice, c'est-à-dire pour un nombre restreint de substances sémiochimiques. De même, le brevet WO2013156249A1 revendique une composition comprenant un pesticide et un copolymère statistique qui contient sous forme polymérisée de l'acide acrylique ou méthacrylique (monomère A), un mono poly(C2-6 alkylène alcool)(méth)acrylate terminé par un alkyle C₁₂-C₂₂ (monomère B), et un méthacrylate d' alkyle C1-C6 (monomère C). Cependant ce type de copolymère statistique est obtenu une fois encore par polymérisation avec un générateur de radical (dans les exemples de ce brevet du tert.-butylperpivalate) qui comme déjà souligné représente un danger de détérioration des substances sémiochimiques courantes. Par exemple, il est bien connu que la (E,Z)-7,9 dodécadiénylacétate (phéromone sexuelle de l'eudémis, tordeuse de la vigne) se transforme en (E,E)-7,9 dodécadiénylacétate qui n'a aucun effet sur l'insecte.

L'état de l'art antérieur illustre bien les difficultés pour l'homme du métier pour trouver des substances solides capables de stocker et relarguer lentement sans modifier la composition chimique des sémiochimiques, en particulier des substances à chaîne grasse, c'est-à-dire lipophiles. Il est essentiel de réussir à créer de tels objets sans utiliser des solvants volatils, des pré-polymères réactifs de type isocyanates, des initiateurs de radicaux libres comme les peroxydes ou des agents de vulcanisation. Par ailleurs, pour contrôler le relargage il faut pouvoir utiliser un matériau capable d'une absorption importante des sémiochimiques, en particulier des substances à chaîne grasse, c'est-à-dire lipophiles, de les protéger contre les rayonnements UV et l'oxygène et de contrôler le relargage sur plusieurs mois.

La présente invention apporte donc une solution pour l'adsorption dans des conditions douces, le stockage et le relargage contrôlé de substances sémiochimiques, en particulier des substances à chaîne grasse, c'est-à-dire lipophiles, répondant aux contraintes ci-dessus.

### DESCRIPTION DE L'INVENTION

La demanderesse a découvert, que de manière surprenante, l'utilisation d'une matrice nanostructurée constituée d'un polymère à base de copolymère à blocs acryliques permet de répondre à la problématique d'absorber, de stocker et de diffuser sur de longues périodes, tout en assurant sa protection, une substance sémiochimique volatile, en particulier une substance sémiochimique volatile à chaîne grasse, c'est-à-dire lipophile.

En effet, ce type de matrice nanostructurée constituée de copolymère à blocs acryliques est généralement utilisée pour ses propriétés adhésives, de résistance au choc, de transparence. Or, de manière surprenante, la demanderesse a découvert qu'elle permettait une absorption quantitative de nombreuses molécules sémiochimiques volatiles, en particulier des substances sémiochimiques volatiles à chaîne grasse, c'est-à-dire lipophiles, à des températures proches de la température ambiante, un stockage et un relargage contrôlé sur de longues durées et qu'elle pouvait être rechargée aisément après utilisation.

Ainsi, dans un premier mode de réalisation, la présente invention concerne une composition comprenant une matrice nanostructurée constituée d'au moins un copolymère à blocs acryliques et une substance sémiochimique volatile, en particulier une substance sémiochimique volatile à chaîne grasse, c'est-à-dire lipophile, dans laquelle le copolymère à blocs acryliques comprend au moins un bloc polyméthacrylate d'alkyle et au moins un bloc poly acrylate d'alkyle.

Ainsi l'invention concerne une composition comprenant une matrice nanostructurée constituée d'au moins un copolymère à blocs acryliques et une substance sémiochimique volatile dans laquelle le copolymère à blocs acryliques comprend au moins un bloc polyméthacrylate d'alkyle et au moins un bloc polyacrylate d'alkyle, caractérisée en ce que la substance sémiochimique volatile est une substance sémiochimique volatile à chaîne grasse, plus particulièrement encore une phéromone à chaîne grasse.

Dans un mode de réalisation particulier la substance sémiochimique volatile est adsorbée à la matrice.

Selon un mode de réalisation, la composition est semi-rigide ou souple.

Selon l'invention, le copolymère à blocs acryliques est un copolymère tri-blocs du type PMMA-PABu-PMMA. La présente invention vise aussi une composition dans laquelle la matrice copolymère comprend 20 à 80% en poids de copolymère à blocs polyméthacrylate d'alkyle et 80 à 20% de copolymère à blocs polyacrylate d'alkyle.

La composition selon l'invention est aussi caractérisée en ce que la substance sémiochimique volatile représente entre 1% et 200% en poids de la composition, particulièrement entre 5 et 150%, plus particulièrement encore entre 5 et 100%, voire plus particulièrement entre 5 et 80%, plus particulièrement encore entre 5 et 50%.

Selon un mode de réalisation de l'invention la substance sémiochimique volatile, en particulier une substance sémiochimique volatile à chaîne grasse, est choisie dans le groupe des phéromones. De manière particulièrement préférée, la substance sémiochimique volatile est lipophile et est une phéromone à chaine grasse.

La substance sémiochimique volatile, en particulier à chaîne grasse, peut être une phéromone sexuelle d'insectes.

Dans un mode de réalisation de la composition selon la présente invention la substance sémiochimique volatile est une phéromone sexuelle de mammifères.

Dans un mode de réalisation de la composition selon la présente invention la substance sémiochimique volatile est une phéromone à chaîne grasse, donc lipophile.

La composition selon l'invention est préférentiellement sous la forme de bloc ou particule solide de forme et de dimension variées.

C'est aussi un autre objet de la présente invention que de fournir une composition selon l'invention telle que décrite ici qui se présente sous la forme d'une ou d'une pluralité de particules solides discrètes rigides, semi-rigides ou souples.

La ou les particules solides discrètes peut être sous la forme de granulé, bloc, pellet, billes par exemple dont la taille et le volume ne sont pas critiques. La composition peut aussi se présenter sous la forme de fil ou pluralité de fils.

C'est aussi un objet de la présente invention que de viser l'utilisation d'une composition selon l'invention pour l'adsorption et le relargage d'une substance sémiochimique, en particulier une substance sémiochimique volatile à chaîne grasse telle qu'une phéromone à chaine grasse, c'est-à-dire lipophile.

L'invention vise ainsi l'utilisation d'une composition selon la présente invention pour la lutte contre les ravageurs des cultures.

Dans un autre mode de réalisation l'invention vise aussi l'utilisation d'une composition selon la présente invention pour le contrôle du comportement des mammifères.

En fin la présente invention vise aussi un procédé de fabrication d'une composition selon l'invention telle que décrite ci-avant comprenant les étapes suivantes :
- Fournir une matrice nanostructurée constituée d'au moins un copolymère à blocs acryliques comprenant au moins un bloc polyméthacrylate d'alkyle et au moins un bloc polyacrylate d'alkyle,
- Mettre en contact ladite matrice avec une substance sémiochimique volatile pendant un temps et à une température permettant l'imprégnation de ladite matrice par ladite substance,
- Récupérer la composition de matrice nanostructurée imprégnée de substance sémiochimique.

Dans le cadre de la présente invention, l'expression « polymère à blocs » représente un polymère dont les macromolécules sont constituées de blocs enchaînés linéairement. Les blocs peuvent être assemblés directement ou par l'intermédiaire d'une unité constitutive qui ne fait pas partie intégrante des blocs.

Dans le cadre de la présente invention, l'expression « copolymère à blocs » : représente un copolymère à blocs issu de plusieurs espèces de polymères.

Dans le cadre de la présente invention, l'expression « bloc » représente la partie d'une macromolécule comprenant une pluralité d'unités constitutives et qui possède au moins une particularité de constitution ou de configuration qui n'apparaît pas dans les parties adjacentes.

L'expression « substance sémiochimique » qualifie une substance chimique émise par une plante ou un animal dans l'environnement et qui a valeur de signal entre les êtres vivants.

Les substances sémiochimiques sont classées en phéromones - qui permettent la communication entre des individus de la même espèce - et allomones, kairomones et synomones qui sont échangées entre des animaux ou des plantes appartenant à des espèces différentes. Les substances sémiochimiques peuvent être perçues par l'odorat pour les composés volatils, ou par le goût pour les composés non volatils. Les informations portées par les sémiochimiques peuvent permettre la localisation et la reconnaissance d'un partenaire sexuel, par exemple.

Les substances sémiochimiques et plus particulièrement les phéromones sont des composés chimiques dont un grand nombre d'exemples (8000) est donné dans la base de données accessible en ligne Pherobase (www.pherobase.com).

Dans la cadre de la présente invention, la substance sémiochimique volatile est une phéromone à chaîne grasse, donc lipophile.

Par l'expression « chaîne grasse », il est entendu au sens de la présente invention, un chaîne aliphatique hydrocarbonnée linéaire ou ramifiée, éventuellement insaturée, comportant 5 à 18, 6 à 16 ou 5 à 16 atomes de carbone, conférant ainsi un caractère lipophile à la substance sémiochimique.

L'expression « volatile » fait référence à la grande volatilité de la substance sémiochique qui peut passer facilement de l'état liquide àl'état gazeux, dans les conditions ambiantes de pression et de température, c'est-à-dire environ 20°C et 760 mm de Hg.

Parmi les exemples de phéromones à chaîne grasse, des phéromones connues et utilisables selon la présente invention sont : les alkanols et alkénols volatiles ayant de 5 à 18 carbones, les alkanals et alkénals volatiles ayant de 5 à 18 carbones, les alkanones ayant de 6 à 18 carbones, le 1,7-dioxaspirononane, le 3-ou 4-hydroxy-1,7-dioxaspiro-undecane, l'alcool benzylique, le Z-(9)-tricosène (muscalure), héneicosène, les acides diacetyl ou alcanoïques ayant de 5 to 16 carbones tels que l' acide caprylique, l'acide laurylique , le pinène, le méthyleugenol, l' éthyldodécanoate, le tert-butyl 4-(or 5-)chloro-2-éthylcyclohexane-carboxylate, la mycrénone, la cucurbitacine, le trimédlure et le (E,E)-8,10-dodécadien-1-ol (codlémone).

De manière avantageuse la substance semiochimique volatile peut être choisie parmi le ferruginéol, la ferruginéone ou un mélange ferruginéol-ferruginéone.

D'autres exemples de phéromones à chaîne grasse connues sont : le Z-5-Décényl acétate, le dodécanyl acétate, le Z-7-dodécényl acetate, le E-7-dodécényl acetate, le Z-8-dodécényl acetate, le E-8-dodécényl acetate, le Z-9-dodécényl acétate, le E-9-dodécényl acétate, le E-10-dodécényl acétate, le 11-dodécényl acétate, le Z- 9,11-dodécadiényl acétate, le E-9,11-dodécadiényl acétate, le Z-11-tridécényl acétate, le E-11-tridécényl acétate, le tétradécényl acétate, le E-7-tétradécényl acétate, le Z-8-tétradécényl acétate, le E-8-tetradacényl acétate, le Z-9-tétradécényl acétate, le E-9-tétradécényl acétate, le Z-10-tétradécényl acétate, le E-10-tétradécényl acétate, le Z-11-tétradécényl acétate, le E-11-tétradécényl acétate, le Z-12- pentadécényl acétate, le E-12-pentadécényl acétate, l'hexadécanyl acétate, le Z-7-hexadécényl acétate, le Z-11-hexadécényl acétate, le E-11-hexadécényl acétate, l'octadécanyl acétate, le E,Z-7,9-dodécadiényl acétate, le Z,E-7,9-dodécadiényl acetate, le E,E-7,9-dodécadiényl acétate, le Z,Z-7,9-dodécadiényl acétate, le E,E-8,10-dodécadienyl acetate, le E,Z-9,12-dodécadiényl acétate, le E,Z-4,7- tri-décadiényl acétate, le 4-méthoxy-cinnamaldéhyde, la [béta]-ionone, l'estragol, l'eugenol, l' indole, le 8-méthyl-2-décyl propanoate, le E,E-9,11-tétradécadiényl acétate, le Z,Z-9,12-tétradécadiényl acétate, le Z,Z-7,11-héxadécadiényl acétate, le E,Z-7,11-héxadécadiényl acetate, le Z,E-7,11-hexadécadiényl acétate, le E,E-7,11-hexadécadiényl acétate, le Z,Z-11,13-hexadécadiényl acétate le Y,Z-11,13-hexadécadiényl acetate, le Z,E-3,13-octadécadiényl acétate, le E,Z-3,13-octadécadiényl acétate, le E,E-3,13-octadécadiényl acétate, l'hexanol, l'heptanol, l'octanol, le décanol, le Z-6-nonénol, le E-6-nonénol, le 4méthyl 5nonanol, la 4 méthyl 5 nonanone, le dodécanol, le 11-dodécénol, le Z-7-dodécénol, le E-7-dodécénol, le Z-8-dodécénol, le E-8-dodécénol, le E-9-dodécénol, le Z-9-dodécénol, le E-9,11-dodécadiénol, le Z-9,11-dodécadiénol, le Z,E-5,7-dodécadiénol, le E,E-5,7-dodécadiénol, le E,E-8,10-dodécadiénol, le E,Z-8,10-dodécadiénol, le Z,Z-8,10-dodécadiénol, le Z,E-8,10-dodécadiénol, le E,Z-7,9-dodécadiénol, le Z,Z-7,9-dodécadiénol, le E-5-tétradécénol, le Z-8-tétradécénol, le Z-9-tétradécénol, le E-9-tetradécénol, le Z-10- tétradécénol, le Z-11-tétradécénol, le E-11-tétradécénol, le Z-11-hexadécénol, le Z,E-9, 11-tétradécadiénol, le Z,E-9,12-tétradécadiénol, le Z,Z-9,12-tétradécadiénol, le Z,Z-10,12-tetradécadiénol, le Z,Z-7,11-hexadécadiénol, Z,E-7,11-hexadecadiénol, (E)-14-méthyl-8-hexadécén-1-ol, le (Z)-14-méthyl-8-hexadécén-1-ol, le E,E-10,12-hexadécadiénol, le E,Z-10,12-hexadécadiénol, le dodécanal, le Z-9-dodécénal, le tétradécanal, le Z-7-tétradécénal, le Z-9-tétradécénal, le Z-11-tétradécénal, le E-11-tétradécénal, le E-11,13-tétradécadiénal, le E,E-8,10-tétradécadiénal, le Z, E-9,11-tétradécadiénal, le Z, E-9,12-tétradécadiénal, l'hexadécanal, le Z-8-hexadécénal, le Z-9-hexadécénal, le Z-10-hexadécénal, le E-10-hexadécénal, le Z-11-hexadécénal, le E-11-hexadécénal, le Z-12-hexadécénal, le Z-13-hexadécénal, le (Z)-14-méthyl-8-hexadécénal, le (E)-14-méthyl-8-hexadécénal, le Z,Z-7, 11-hexadécadiénal, le Z,E-7,11-hexadécadiénal, le Z,E-9, 11-hexadécadiénal, le E,E-10, 12-hexadécadiénal, le E,Z-10, 12-hexadécadiénal, le Z, E-10,12-hexadécadiénal, le Z,Z-10,12-hexadécadiénal, le Z,Z-11, 13-hexadécadiénal, l'octadécanal, le Z-11-octadécénal, le E-13-octadécénal, le Z-13-octadécénal, le Z-5-décényl-3-méthyl butanoate, la disparlure: (+) cis-7,8-epoxy-2-methyloctadecane, le seudénol: 3- methyl-2-cyclohexen-1-ol, le sulcatol: 6-methyl-5-hepten-2-ol, l'ipsénol: 2-methyl-6-methylene-7- octen-4-ol, l'ipsdiénol: 2-methyl-6-methylene-2, 7-octadien-4-ol, la grandiure I : cis-2-isopropenyl-1-methylcyclobutane-ethanol, la grandiure II : Z-3,3-dimethyl-1-cyclohexane-ethanol, la grandiure III : Z-3,3-dimethyl-1-cyclohexane-acataldéhyde, la grandlure IV: E-3,3-diméthyl-1-cyclohexane- acétaldéhyde, cis-2-ver-benol: cis-4,6,6-triméthylbicyclo[3, 1,1]hept-3-en-2-ol , la cucurbitacine, 2- méthyl-3-buten-2-ol, 4-méthyl-3-heptanol, la cucurbitacine, 2-méthyl-3-buten-2-ol, 4-méthyl-3- heptanol, l'[alpha]-pinène: 2,6,6-triméthylbicyclo[3, 1,1]hepten-2-ène, l'[alpha]-caryophyllène: 4, 11,11-triméthyl-8-méthylène-bicyclo[7,2,0]undécane, le Z-9-tricosène, l'[alpha]-multistriatine, le 2-(2-endo,4-endo)-5-éthyl-2,4-diméthyl-6,8-dioxabicyclo [3,2,1] octane, le méthyleugénol: 1,2- dimethoxy-4-(2-propenyl)phenol, la linéatine: 3,3,7-triméthyl-2,9-dioxatricyclo [3,3, 1,0] nonane, le chalcogran : 2-éthyl-1,6-dioxaspiro[4,4]nonane, la frontaline : 1,5-diméthyl-6,8-dioxabicyclo[3,2, 1]octane, l'endo-brevicomine : endo-7-éthyl-5-méthyl-6,8-dioxabicyclo[3,2,1]octane, l'exo-brévicomine : exo-7-éthyl-5-méthyl-6,8-dioxabicyclo[3,2,1]octane, la (Z)-5-(1-décényl)dihydro-2-(3H)-furanone, le farnésol : 3,7,11-triméthyl-2,6, le 10-dodécatrien-1-ol, le nérolidol 3,7-11-triméthyl-1,6,10-dodécatrién-3-ol, le 3-méthyl,6-(1-méthyléthényl)-9-décen-1-ol acétate, le (Z)-3-méthyl-6-(1-méthyléthényl)-3, le 9-décadién-1-ol acétate, le (E)-3,9-méthyl-6-(1-méthyl-éthényl)-5,8-décadién-1-ol acétate, le 3- méthylène-7-méthyl-octen-1-ol propionate, le (Z)-3,7-diméthyl-2,7-octadién-1-ol le propionate, (Z)- 3,9-diméthyl-6-(1-méthyl-éthényl)-3,9-décadlién-1-ol.

Comme indiqué, selon un mode de réalisation, la substance sémiochimique est choisie dans le groupe des phéromones, en particulier parmi les phéromones sexuelles d'insectes, en particulier les phéromones sexuelles d'insectes à chaîne grasse, et plus particulièrement encore dans le groupe constitué par les phéromones des papillons des processionnaires du pin, les phéromones des papillons des processionnaires du chêne, les phéromones de la pyrale du buis, les phéromones des bombyx, les phéromones des carpocapses, les phéromones de la mouche du pêcher, les phéromones de la sésamie du maïs, les phéromones de l'eudémis, les phéromones de la batrachédra, les phéromones de la miride du cacaoyer, les phéromones des charançons des palmiers ou du bananier, les phéromones de l'eudémis et les phéromones de la cochylis de la vigne ainsi que leurs mélanges.

Selon une autre mode de réalisation de l'invention la substance sémiochimique **volatile** est choisie dans le groupe des substances sémiochimiques de mammifères, en particulier les phéromones de mammifères, plus particulièrement les phéromones de mammifères à chaîne grasse, et plus particulièrement encore les phéromones des mammifères de type acides ou esters gras choisi dans le groupe constitué par le méthyl laurate, les alkylglycérols (AKG) ou leurs dérivés et le 2-méthyl but-2-énal ainsi que leurs mélanges. Parmi ces phéromones animales, on peut par exemple citer : les phéromones apaisantes telles que la fraction F3 (phéromone faciale) pour le chat obtenue selon le brevet EP0724832B1, ou les phéromones mimant les phéromones naturelles de la femelle allaitante telles que décrite dans WO9937297 (par exemple commercialisée dans les produits tels que Adaptil®, Suilance®), ou les compositions contenant des alkyl glycérols tels que l'alcool érucique, l'alcool chimilique ou leurs acétals obtenus avec le 2-méthyl but-2-énal.

Un avantage de l'invention consiste d'abord en la simplicité du procédé de préparation des compositions selon l'invention qui comprend un simple mélange de la substance sémiochimique avec la matrice polymère sous quelle forme que ce soit à une température de l'ordre de 20 à 40°C pendant une durée suffisante pour assurer l'adsorption de ladite substance sur ladite matrice. Typiquement la durée de ce mélange est de quelques minutes à plusieurs heures, plus particulièrement environ 1 heure. Le mélange peut être réalisé par tout dispositif adéquat de type mélangeur de laboratoire ou autre de type tel que ceux utilisés pour la dragéification par exemple.

Il est notable de remarquer que l'imprégnation et l'adsorption de la substance par la matrice ne nécessite aucun solvant et est réalisée par la simple mise en contact des deux.

La matrice copolymère selon l'invention présente une bonne tenue mécanique, signe que les blocs de poly(méthacrylate de méthyle) jouent un rôle dans l'organisation de la matrice polymère. Sans être lié par une quelconque théorie, il semblerait donc que la présence des blocs acrylates facilite une absorption rapide des substances sémiochimiques par le copolymère.

La nature nanostructurée des copolymères est importante. Elle traduit la présence de domaines de polarités très différentes à température ambiante (environs 20°C) : l'une solide (polyméthacrylate d'alkyle, plus particulièrement PMMA) qui est imperméable aux substances sémiochimiques telles que les phéromones mais assure la tenue mécanique de la matrice ; l'autre liquide fondu (polyacrylate d'alkyle, plus particulièrement Polyacrylate de butyle par exemple) qui solubilise et permet la mobilité de la substance sémiochimique volatile telle qu'une phéromone. En absence de la nanostructuration, l'absorption de la substance sémiochimique, induirait une liquéfaction du polymère ce qui n'est pas souhaitable pour des objets solides rigides, semi-rigides ou souples.

La composition nanostructurée donne lieu à une structure co-continue de la phase bloc polyacrylate d'alkyle (ie liquide fondu à température ambiante) créant ainsi des canaux de pénétration (avec par exemple au moins 30% de blocs acrylate) pour que la substance sémiochimique volatile telle qu'une phéromone, en particulier une phéromone à chaîne grasse, puisse diffuser et s'imprégner dans le matériau.

La forme de la composition n'a pas d'importance pour l'imprégnation. Cette imprégnation peut être réalisée sur des billes (granulés) ou des fils de copolymère. La nature co continue fait que le matériau se gonfle de substance sémiochimique volatile, par exemple une phéromone, comme une éponge.

La nature nanostructurée peut se caractériser de multiples manières : par exemple par imagerie microscopique électronique, par mesure de viscosité en fonction de la température (on voit dès lors apparaitre des transitions vitreuses correspondant à chaque bloc pur du copolymère). Il est probable que la grande similarité chimique entre un ester acrylique et par exemple une phéromone telle qu'une phéromone à chaine grasse de lépidoptère soit la cause de la rapidité d'imprégnation du matériau par la phéromone et expliquerait qu'aucun solvant ne soit nécessaire pour faciliter cette imprégnation.

Des matrices nanostructurées constituée de copolymères à blocs acryliques convenables pour l'invention sont bien décrites dans « Enhanced toughness properties in nanostructured glass polymers by all-acrylic block copolymers prepared by Controlled Radical polymerization » (P. GERARD, O. GUERRET - ANTEC. Conférence proceedings, 2004 2004, vol. 2, pp. 2004-2008. Society of Plastics Engineers, Brookfield, CT, ETATS-UNIS (1983-2004).

Différentes matrices de copolymères à blocs sont disponibles dans le commerce auprès de la société Arkema, elles sont par exemple constituées de copolymères Nanostrengh® triblock MAM-ABu-MAM de poly(acrylate de butyle) (PABu) et de poly(méthacrylate de méthyle)(PMMA) obtenus par polymérisation radicalaire contrôlée par un nitroxyde selon la technologie connue et documentée Blockbuilder®.

Dans un mode de réalisation particulier de l'invention, la substance sémiochimique pourra être protégée contre l'action de la lumière et/ou celle de l'oxygène. En effet, lorsque les diffuseurs sont utilisés dans des cultures pendant plusieurs mois, il pourra être préférable de protéger la substance sémiochimique d'une altération dans le temps. L'homme du métier a l'habitude d'additionner des antioxydants et des anti-UV aux substances sémiochimiques pour remplir cette fonction. Un avantage du procédé selon l'invention est que les stabilisants UV ou les antioxydants peuvent être déjà contenus dans la matrice polymère avant son imprégnation ou bien ajoutés au sémiochimique avant que celui-ci ne soit incorporé/adsorbé dans/par la matrice polymère. Ces deux méthodes assurent une protection intime du sémiochimique jusqu'à sa diffusion dans le milieu extérieur.

Les antioxydants peuvent être choisis dans le groupe constitué par la vitamine E, l'hydroxyanisole butylé(BHA), l'hydroxytoluène butylé (BHT) utilisés seuls ou en mélange. Ces antioxydant protègent la substance sémiochimique de la dégradation et peuvent être ajoutés dans des quantités, en pourcentage en poids de la composition, allant de 0,1% environ à 3% environ, particulièrement entre 0.5 et 2%.

Les additifs anti-UV peuvent être choisis dans le groupe constitué par le béta-carotène, l'acide p-aminobenzoïque, les amines encombrées et les alkoxyamines encombrées utilisés seuls ou en mélange. Ces anti-UV protègent les sémiochimques de la dégradation par la lumière et peuvent être ajoutés dans des quantités, en pourcentage en poids de la composition, allant de 0,1% environ à 3% environ, particulièrement entre 0.5 et 2%.

Un autre avantage de la composition selon l'invention est qu'elle peut se réaliser sur toute forme d'objet. Ainsi il est possible de transformer, par injection par exemple, la matrice copolymère à bloc en un objet solide tel qu'un collier, une plaquette, un crochet en rapport avec le mode d'utilisation du diffuseur. Ces objets ainsi formés peuvent ensuite être imprégnés par le sémiochimique selon le procédé décrit ci-dessus.

Un autre avantage particulièrement important de la composition selon l'invention est que le matériau peut être rechargé. En effet, une fois que la quantité initiale de composé sémiochimique a été relarguée et diffusée, il est possible de récupérer la matrice polymère (quelle que soit sa forme) et de la ré-imprégner de sémiochimique selon le procédé décrit ci-dessus. Un tel avantage est très important car il permet de recycler les objets ce qui a un sens tant économique qu'écologique.

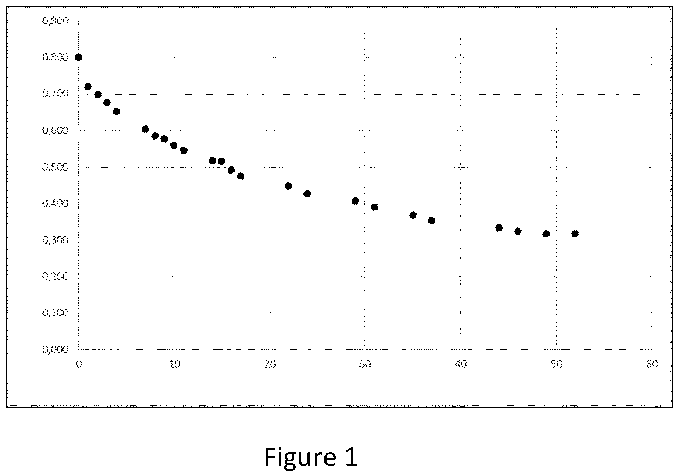

FIGURE 1 : Suivi de la cinétique de relargage par une composition contenant 8,8g de matrice copolymère à blocs M53 chargée à 9,1% en phéromone du charançon du palmier (soit 800mg) a été effectué suivant le protocole décrit précédemment dans une étuve ventilée à 30 °C. En ordonnée, est représentée la masse de phéromone restante (en grammes) en fonction de la durée en jours figurée en abscisse. Après 50 jours, 60% de la quantité de phéromone absorbée a diffusé soit environ 500mg de phéromone

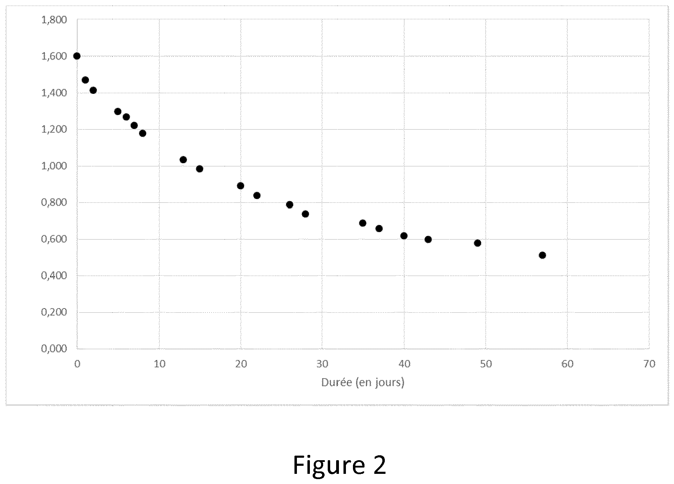

FIGURE 2 : Suivi de la cinétique de relargage par une composition contenant 8,8g de matrice copolymère à blocs M53 chargé à 18,2% en phéromone du charançon du palmier (soit 1,6g) est fait selon la procédure décrite précédemment dans une étuve ventilée à 30 °C. En ordonnée, est représentée la masse de phéromone restante (en grammes) en fonction de la durée en jours figurée en abscisse. Après 55 jours, environ 63% de la quantité de phéromone absorbée a diffusé soit environ 1 g de phéromone

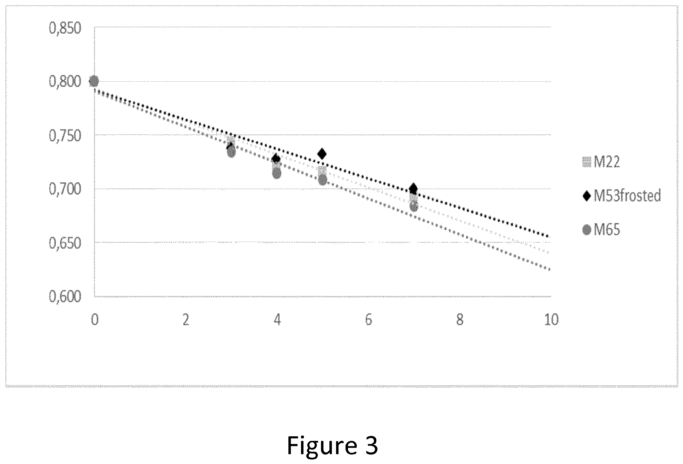

FIGURE 3 : Cinétique de diffusion comparative du ferruginéol dans différents types de copolymères à blocs : Carrés gris (M22) ; Lozenges noirs (M53 frosted) et Ronds gris (ronds gris). En ordonnée, est représentée la masse de phéromone restante (en grammes) en fonction de la durée en jours figurée en abscisse.

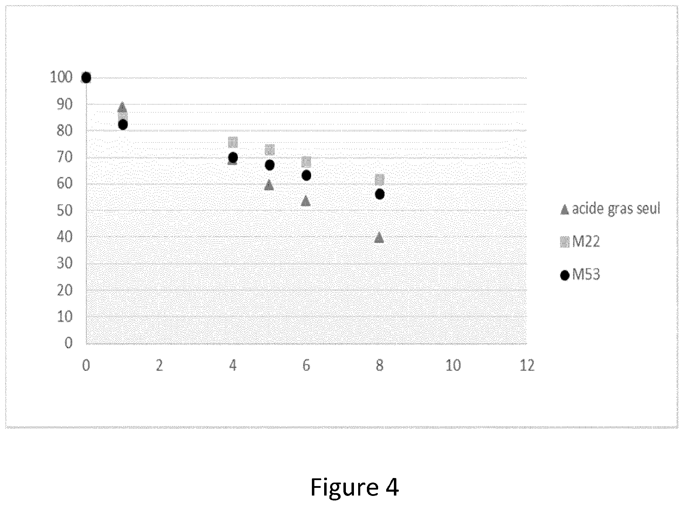

FIGURE 4 : Etude de diffusion du méthyl laurate seul (triangles gris) et à 5% dans les matrices copolymères à bloc M22 (carrés gris) et M53 (ronds noirs). En ordonnée, est représentée la masse de phéromone restante (en grammes) en fonction de la durée en jours figurée en abscisse.

### EXEMPLES

### Mode opératoire général d'obtention de la composition par absorption de phéromones dans la matrice de copolymères à blocs acryliques

Les différentes matrices de copolymères à blocs sont achetées à la société Arkema, elles sont constituées de copolymères Nanostrengh® triblock MAM-ABu-MAM de poly(acrylate de butyle) (PABu) et de poly(méthacrylate de méthyle)(PMMA) obtenus par polymérisation radicalaire contrôlée par un nitroxyde Blockbuilder®. Plusieurs matrices copolymères ont été utilisées, elles possèdent les caractéristiques suivantes :

| **Composition** | **PABu** | **PMMA** |
|---|---|---|
| Nom générique de la matrice copolymère nanostrength® | | |
| M22 | 40 | 60 |
| M53 | 50 | 50 |
| M53 frosted | M53+ajout de PMMA microsphère | |
| M65 | 62 | 38 |

Les substances sémiochimiques volatiles à chaîne grasse, principalement des mélanges phéromonaux à chaîne grasse, sont soit achetées chez des fabricants industriels soit fabriquées par M2i Development (Lacq, France) selon des procédés connus. Dans le cas des produits synthétisés par M2i Development, leurs caractérisations sont validées par une analyse comparative avec un échantillon référent en chromatographie phase gazeuse.

Dans un réacteur muni d'un agitateur mécanique sont ajoutés une masse donnée de granulés de copolymères à bloc. L'agitateur est alors mis en marche et le milieu est chauffé à 40 °C. La substance sémiochimique est alors ajoutée goutte à goutte et le mélange est agité pendant 1h. Après refroidissement, les copolymères à blocs imprégnés de la substance sémiochimique sont prélevés. La composition se présente sous la forme de granulés imprégnés. Leur masse après séchage est mesurée pour évaluer le taux d'absorption de la substance sémiochimique dans et sur la matrice copolymère par différence avec la masse initiale de matrice copolymère.

Pour étudier le relargage de la substance sémiochimique, une quantité donnée de la composition (granulés imprégnés de la substance sémiochimique) est placée dans une enceinte ventilée à 30°C (condition correspondant à une température ambiante). La composition est pesée à différents temps sur des durées longues et les courbes de relargage en sont déduites.

### Exemple 1 : Imprégnation à 9,1% de mélange phéromonal du charançon rouge des palmiers (Rhynchophorus ferrugineus) sur copolymères à blocs M53.

Dans un réacteur muni d'un agitateur mécanique sont ajoutés 90.0g de billes de copolymères à bloc M53. L'agitateur est alors mis en marche et le milieu est chauffé à 40 °C. 9.0g du mélange ferruginéol-ferruginéone (9-1) sont alors ajoutés goutte à goutte. Le milieu est ensuite agité pendant 1h. Après refroidissement, les copolymères à blocs sont prélevés et pesés avant d'être conditionnés. Le taux d'imprégnation de la matrice copolymère par le mélange ferruginéol-ferruginéone est estimé à 9,1%.

### Exemple 2 : Imprégnation à 18,2% de mélange phéromonal du charançon rouge des palmiers (Rhynchophorus ferrugineus) sur copolymères à blocs M53.

Dans un réacteur muni d'un agitateur mécanique sont ajoutés 90.0g de billes de copolymères à bloc M53. L'agitateur est alors mis en marche et le milieu est chauffé à 40 °C. 18 g du mélange ferruginéol-ferruginéone (9-1) sont alors ajoutés goutte à goutte. Le milieu est ensuite agité pendant 1h. Après refroidissement, les copolymères à blocs sont prélevés et pesés avant d'être conditionnés. Le taux d'imprégnation de la matrice copolymère par le mélange ferruginéol-ferruginéone est estimé à 18,2%.

### Exemple 3 : Saturation des copolymères à blocs M53 avec le mélange phéromonal du charançon rouge des palmiers (Rhynchophorus ferrugineus).

Dans un réacteur muni d'un agitateur mécanique sont ajoutés 90.0g de billes de copolymères à bloc M53. Le mélange ferruginéol-ferruginéone (9-1) est ajouté goutte à goutte jusqu'à recouvrement des billes de polymère. L'agitateur est alors mis en marche et le milieu est chauffé à 40 °C. Le milieu est ensuite agité pendant 1h. Après refroidissement, les copolymères à blocs sont filtrés puis prélevés et pesés avant d'être conditionnés. Le taux d'imprégnation à saturation de la matrice copolymère M53 par le mélange ferruginéol-ferruginéone est estimé à 26%.

### Exemple 4 : Imprégnation à 9,1% de mélange phéromonal du charançon rouge des palmiers (Rhynchophorus ferrugineus) sur copolymères à blocs M22.

Dans un réacteur muni d'un agitateur mécanique sont ajoutés 90.0g de billes de copolymères à bloc M22. L'agitateur est alors mis en marche et le milieu est chauffé à 40 °C. 9 g du mélange ferruginéol-ferruginéone (9-1) sont alors ajoutés goutte à goutte. Le milieu est ensuite agité pendant 1h. Après refroidissement, les copolymères à blocs sont prélevés et pesés avant d'être conditionnés. Le taux d'imprégnation de la matrice copolymère par le mélange ferruginéol-ferruginéone est estimé à 9,1%.

### Exemple 5 : Imprégnation à 9,1% de mélange phéromonal du charançon rouge des palmiers (Rhynchophorus ferrugineus) sur copolymères à blocs M65.

Dans un réacteur muni d'un agitateur mécanique sont ajoutés 90.0g de billes de copolymères à bloc M65. L'agitateur est alors mis en marche et le milieu est chauffé à 40 °C. 9 g du mélange ferruginéol-ferruginéone (9-1) sont alors ajoutés goutte à goutte. Le milieu est ensuite agité pendant 1h. Après refroidissement, les copolymères à blocs sont prélevés et pesés avant d'être conditionnés. Le taux d'imprégnation de la matrice copolymère par le mélange ferruginéol-ferruginéone est estimé à 9,1%.

### Exemple 6 : Imprégnation à 9,1% de mélange phéromonal du charançon rouge des palmiers (Rhynchophorus ferrugineus) sur copolymères à blocs M53 frosted.

Dans un réacteur muni d'un agitateur mécanique sont ajoutés 90.0g de billes de copolymères à bloc M53 frosted. L'agitateur est alors mis en marche et le milieu est chauffé à 40 °C. 9 g du mélange ferruginéol-ferruginéone (9-1) sont alors ajoutés goutte à goutte. Le milieu est ensuite agité pendant 1h. Après refroidissement, les copolymères à blocs sont prélevés et pesés avant d'être conditionnés. Le taux d'imprégnation de la matrice copolymère par le mélange ferruginéol-ferruginéone est estimé à 9,1%.

### Exemple 7 : Imprégnation de la phéromone d'agrégation du charançon du bananier (Cosmopolites sordidus) sur copolymères à blocs M53

Dans un réacteur muni d'un agitateur mécanique sont ajoutés 30.0g de billes de copolymères à bloc « M53 frosted ». L'agitateur est alors mis en marche et le milieu est chauffé à 40 °C. 309 mg de sordidine sont alors ajoutés goutte à goutte. Le milieu est ensuite agité pendant 1h. Après refroidissement, les copolymères à blocs sont prélevés et pesés avant d'être conditionnés. Le taux d'imprégnation de la matrice copolymère par la sordidine est estimé à 1%.

### Exemple 8 : Imprégnation de la phéromone d'agrégation du charançon du bananier (Cosmopolites sordidus) sur copolymères à blocs M22

Dans un réacteur muni d'un agitateur mécanique sont ajoutés 30.0g de billes de copolymères à bloc M22. L'agitateur est alors mis en marche et le milieu est chauffé à 40 °C. 309mg de sordidine sont alors ajoutés goutte à goutte. Le milieu est ensuite agité pendant 1h. Après refroidissement, les copolymères à blocs sont prélevés et pesés avant d'être conditionnés. Le taux d'imprégnation de la matrice copolymère par la sordidine est estimé à 1%.

### Exemple 9 : Imprégnation du méthyl laurate, ester gras volatile qui entre dans la composition des phéromones apaisantes pour les mammifères sur copolymères à blocs M53.

Dans un réacteur muni d'un agitateur mécanique sont ajoutés 9,5g de billes de copolymères à bloc M53. L'agitateur est alors mis en marche et le milieu est chauffé à 40 °C. 506mg de méthyl laurate sont alors ajoutés goutte à goutte. Le milieu est ensuite agité pendant 15'. Après refroidissement, les copolymères à blocs sont prélevés et pesés avant d'être conditionnés. Le taux d'imprégnation de la matrice copolymère par le mélange méthyl laurate est estimé à 5%.

### Exemple 10 : Imprégnation du méthyl laurate sur copolymères à blocs M22

Dans un réacteur muni d'un agitateur mécanique sont ajoutés 9,5g de billes de copolymères à bloc M22. L'agitateur est alors mis en marche et le milieu est chauffé à 40 °C. 506mg de méthyl laurate sont alors ajoutés goutte à goutte. Le milieu est ensuite agité pendant 15'. Après refroidissement, les copolymères à blocs sont prélevés et pesés avant d'être conditionnés. Le taux d'imprégnation de la matrice copolymère par le mélange méthyl laurate est estimé à 5%.

### Exemple 11 : Imprégnation de la phéromone de la processionnaire du pin sur copolymères à blocs M53

Dans un réacteur muni d'un agitateur mécanique sont ajoutés 10.0g de billes de copolymères à bloc M53. L'agitateur est alors mis en marche et le milieu est chauffé à 40 °C. 1.06 g de la phéromone de la processionnaire du pin (Y,Z)(11, 13)hexadecadienyl acétate) sont alors ajoutés goutte à goutte. Le milieu est ensuite agité pendant 1h. Après refroidissement, les copolymères à blocs sont prélevés et pesés avant d'être conditionnés. 976 mg de phéromone s'est imprégnée sur les billes, c'est-à-dire une imprégnation à 8.89%.

### Exemple 12 : Imprégnation de la phéromone du ver de la grappe (eudemys) sur copolymères à blocs M53

Dans un réacteur muni d'un agitateur mécanique sont ajoutés 10.1g de billes de copolymères à bloc M53. L'agitateur est alors mis en marche et le milieu est chauffé à 40 °C. 1.06g de lobésia ((E,Z)(7,9)-dodecadiényl acétate) sont alors ajoutés goutte à goutte. Le milieu est ensuite agité pendant 1h. Après refroidissement, les copolymères à blocs sont prélevés et pesés avant d'être conditionnés. 824mg de phéromone s'est imprégnée sur les billes, c'est-à-dire une imprégnation à 7.54%.

### Exemple 13 : Imprégnation de la phéromone du carpocapse ravageur de la pomme sur copolymères à blocs M53

Dans un réacteur muni d'un agitateur mécanique est ajouté 1g de ((E,E)8-10dodécanediénol) phéromone du carpocapse. L'agitateur est alors mis en marche et le milieu est chauffé à 40 °C. 10.1g de billes de copolymères à bloc M53 est alors ajouté. Le milieu est ensuite agité pendant 3h. Après refroidissement, les copolymères à blocs sont prélevés et pesés avant d'être conditionnés. Le taux d'imprégnation de la matrice copolymère par la phéromone du carpocapse est estimé à 9 %.

### Exemple 14 : Imprégnation de la phéromone sexuelle de la pyrale du buis (Cydalima perspectalis) sur copolymères à blocs M53

Dans un réacteur muni d'un agitateur mécanique sont ajoutés 7,53g de billes de copolymères à bloc M53. L'agitateur est alors mis en marche et le milieu est chauffé à 40 °C. 752mg du mélange phéromonal [Z11-hexadecenal/E11-hexadecenal (5:1)] est alors ajouté goutte à goutte. Le milieu est ensuite agité pendant 1h. Après refroidissement, les copolymères à blocs sont prélevés et pesés (8,13g) avant d'être conditionnés. Le taux d'imprégnation de la matrice copolymère en phéromone est de 7,4%.

### Exemple 15 : Imprégnation de la fraction phéromonale F3 apaisante des chats sur copolymères à blocs M53

Dans un réacteur muni d'un agitateur mécanique sont ajoutés 10g de billes de copolymères à bloc M53. L'agitateur est alors mis en marche et le milieu est chauffé à 40 °C. 1g du mélange phéromonal F3 (telle que décrite dans EP0724832B1) est alors ajouté goutte à goutte. Le milieu est ensuite agité pendant 1h. Après refroidissement, les copolymères à blocs sont prélevés et pesés avant d'être conditionnés. Le taux d'imprégnation de la matrice copolymère en phéromone est estimé à 7,7%.

### Exemple 16 : Imprégnation d'une phéromone apaisante des chiens sur copolymères à blocs M53

Dans un réacteur muni d'un agitateur mécanique sont ajoutés 10g de billes de copolymères à bloc M53. L'agitateur est alors mis en marche et le milieu est chauffé à 40 °C. 1g du mélange phéromonal (constitué d'esters gras tel que décrit dans WO 9937297) est alors ajouté goutte à goutte. Le milieu est ensuite agité pendant 1h. Après refroidissement, les copolymères à blocs sont prélevés et pesés avant d'être conditionnés. Le taux d'imprégnation de la matrice copolymère en phéromone est estimé à 8,8%.

### Exemple 17 : Imprégnation d'une phéromone apaisante des chevaux sur copolymères à blocs M53

Dans un réacteur muni d'un agitateur mécanique sont ajoutés 10g de billes de copolymères à bloc M53. L'agitateur est alors mis en marche et le milieu est chauffé à 40 °C. 1,02g du mélange phéromonal (acide oléique/AKG-2M2B 97 :3) sont alors ajoutés goutte à goutte. Le milieu est ensuite agité pendant 5h. Après refroidissement, les copolymères à blocs sont prélevés et pesés (10,88g) avant d'être conditionnés. Le taux d'imprégnation de la matrice copolymère en phéromone est estimé à 8,1%.

### Exemple 18 : ré-imprégnation

10 g de billes imprégnées selon l'exemple 2 sont placées dans une coupelle qui est mise dans une étuve ventilée à 60°C pendant 7 jours. On obtient alors 8.65g de billes ce qui signifie que 75% de la phéromone a été relarguée.

Les billes de copolymères sont alors introduites dans un réacteur muni d'un agitateur mécanique. L'agitateur est alors mis en marche et le milieu est chauffé à 40 °C. 1.35 g du mélange ferruginéol-ferruginéone (9-1) sont alors ajoutés goutte à goutte. Le milieu est ensuite agité pendant 1h. Après refroidissement, les copolymères à blocs sont prélevés et pesés avant d'être conditionnés. Le taux d'imprégnation de la matrice copolymère par le mélange ferruginéol-ferruginéone est estimé à 18,1%.

## Revendications

1. Composition comprenant une matrice nanostructurée constituée d'au moins un copolymère à blocs acryliques et une substance sémiochimique volatile dans laquelle le copolymère à blocs acryliques comprend au moins un bloc polyméthacrylate d'alkyle et au moins un bloc polyacrylate d'alkyle, **caractérisée en ce que** la substance sémiochimique volatile est une phéromone à chaîne grasse et que le copolymère à blocs acryliques est un copolymère tri-blocs du type PMMA-PABu-PMMA.

2. Composition selon la revendication 1, **caractérisée en ce que** la matrice copolymère comprend 20 à 80% en poids de copolymère à blocs polyméthacrylate d'alkyle et 80 à 20% de copolymère à blocs polyacrylate d'alkyle.

3. Composition selon l'une des revendications précédentes, **caractérisée en ce que** la substance sémiochimique volatile représente entre 1% et 200% en poids de la composition, particulièrement entre 5 et 150%, plus particulièrement encore entre 5 et 100%, voire plus particulièrement entre 5 et 80%, plus particulièrement encore entre 5 et 50%.

4. Composition selon la revendication 1, **caractérisée en ce que** la substance sémiochimique volatile est une phéromone sexuelle d'insectes.

5. Composition selon la revendication 1, **caractérisée en ce que** la substance sémiochimique volatile est une phéromone sexuelle de mammifères.

6. Composition selon l'une des revendications 1 à 5, **caractérisée en ce qu'**elle se présente sous la forme d'une ou d'une pluralité de particules solides discrètes.

7. Utilisation d'une composition selon l'une des revendications 1 à 6 pour la lutte contre les ravageurs des cultures.

8. Utilisation non thérapeutique d'une composition selon l'une des revendications 1 à 6 pour le contrôle du comportement des mammifères.

9. Procédé de fabrication d'une composition selon la revendication 1 comprenant les étapes suivantes :
- Fournir une matrice nanostructurée constituée d'au moins un copolymère à blocs acryliques comprenant au moins un bloc polyméthacrylate d'alkyle et au moins un bloc polyacrylate d'alkyle,
- Mettre en contact ladite matrice avec une substance sémiochimique volatile pendant un temps et à une température permettant l'imprégnation de ladite matrice par ladite substance,
- Récupérer la composition de matrice nanostructurée imprégnée de substance sémiochimique.

## Patentansprüche

1. Zusammensetzung, die eine nanostrukturierte Matrix umfasst, die aus mindestens einem Copolymer mit Acrylblöcken und einer flüchtigen Semiochemikalie besteht, wobei das Copolymer mit Acrylblöcken mindestens einen Alkylpolymethacrylatblock und mindestens einen Alkylpolyacrylatblock umfasst, **dadurch gekennzeichnet, dass** die flüchtige Semiochemikalie ein Pheromon mit Fettkette ist und dass das Copolymer mit Acrylblöcken ein Triblock-Copolymer vom Typ PMMA-PABu-PMMA ist.

2. Zusammensetzung nach Anspruch 1, **dadurch gekennzeichnet, dass** die Copolymermatrix 20 bis 80 Gew.-% Copolymer mit Alkylpolymethacrylatblöcken und 80 bis 20 Gew.-% Copolymer mit Alkylpolyacrylatblöcken umfasst.

3. Zusammensetzung nach einem der vorhergehenden Ansprüche, **dadurch gekennzeichnet, dass** die flüchtige Semiochemikalie zwischen 1 und 200 Gew.-%, genauer gesagt zwischen 5 und 150 Gew.-%, noch genauer gesagt zwischen 5 und 100 Gew.-%, genauer gesagt zwischen 5 und 80 Gew.-% und noch genauer gesagt zwischen 5 und 50 Gew.-% der Zusammensetzung darstellt.

4. Zusammensetzung nach Anspruch 1, **dadurch gekennzeichnet, dass** die Semiochemikalie ein Sexualpheromon von Insekten ist.

5. Zusammensetzung nach Anspruch 1, **dadurch gekennzeichnet, dass** die flüchtige Semiochemikalie ein Sexualpheromon von Säugetieren ist.

6. Zusammensetzung nach einem der Ansprüche 1 bis 5, **dadurch gekennzeichnet, dass** sie die Form von einem oder mehreren diskreten festen Partikeln aufweist.

7. Verwendung einer Zusammensetzung nach einem der Ansprüche 1 bis 6 bei der Bekämpfung von Kulturschädlingen.

8. Nichttherapeutische Verwendung einer Zusammensetzung nach einem der Ansprüche 1 bis 6 für die Steuerung des Verhaltens von Säugetieren.

9. Verfahren zur Herstellung einer Zusammensetzung nach Anspruch 1, das die folgenden Schritte umfasst:
- Bereitstellen einer nanostrukturierten Matrix, die aus mindestens einem Copolymer mit Acrylblöcken besteht, die mindestens einen Alkylpolymethacrylatblock und mindestens einen Alkylpolyacrylatblock umfassen,
- Inkontaktbringen der Matrix mit einer flüchtigen Semiochemikalie während einer Zeit und bei einer Temperatur, die die Imprägnierung der Matrix durch die Semiochemikalie ermöglichen,
- Wiedergewinnen der mit der Semiochemikalie imprägnierten nanostrukturierten Matrix.

## Claims

1. A composition comprising a nanostructured matrix consisting of at least one acrylic block copolymer and a volatile semiochemical wherein the acrylic block copolymer comprises at least one poly(alkyl methacrylate) block and at least one poly(alkyl acrylate) block, **characterized in that** the volatile semiochemical is a fatty-chain pheromone and that the acrylic block copolymer is a tri-block copolymer of the PMMA-PABu-PMMA type.

2. The composition according to claim 1, **characterized in that** the copolymer matrix comprises 20 to 80% by weight of poly(alkyl methacrylate) block copolymer and 80 to 20% of poly(alkyl acrylate) block copolymer.

3. The composition according to one of the preceding claims, **characterized in that** the volatile semiochemical represents between 1% and 200% by weight of the composition, particularly between 5 and 150%, more particularly between 5 and 100%, even more particularly between 5 and 80%, more particularly still between 5 and 50%.

4. The composition according to claim 1, **characterized in that** the volatile semiochemical substance is an insect sex pheromone.

5. The composition according to claim 1, **characterized in that** the volatile semiochemical is a mammalian sex pheromone.

6. The composition according to one of claims 1 to 5, **characterized in that** it is in the form of one or a plurality of discrete solid particles.

7. Use of a composition according to one of claims 1 to 6 to control crop pests.

8. Non-therapeutic use of a composition according to one of claims 1 to 6 to control mammalian behaviour.

9. A method for producing a composition according to claim 1, comprising the following steps:
- Providing a nanostructured matrix consisting of at least one acrylic block copolymer comprising at least one poly(alkyl methacrylate) block and at least one poly(alkyl acrylate) block,
- Bringing said matrix into contact with a volatile semiochemical for a period of time and at a temperature allowing the impregnation of said matrix by said semiochemical,
- Recovering the semiochemical-impregnated nanostructured matrix composition.
